# EUROPEAN PATENT APPLICATION

(11) **EP 4 643 898 A1**
(43) Date of publication of application: **05.11.2025**
(21) Application number: 25172943.0
(22) Date of filing: 28.04.2025
(51) Int. Cl.: A61M 1/16, A61M 1/34, A61M 1/36

(54) **BLOOD PURIFICATION APPARATUS**

(30) Priority: 30.04.2024 JP 2024074129
(71) Applicant: Shibuya Corporation, Kanazawa-shi, Ishikawa 920-8681 (JP)
(72) Inventor: TAKEMURA, Hideo, Ishikawa, 920-8681 (JP); MATSUBARA, Yosuke, Ishikawa, 920-8681 (JP)
(74) Representative: Mewburn Ellis LLP

(57) **Abstract**

An object is to efficiently perform a priming procedure before treatment and a blood returning procedure after the treatment. Provided are: a fluid supply passage 11 which is connected at one end to a dialysis fluid supply passage 4A in a dialysis fluid circuit and which is connected at the other end to a vein passage 3A in a blood circuit; and a supply/discharge passage 12 which is connected at one end to an artery passage 3B in the blood circuit and which is connected at the other end while being able to switch between the dialysis fluid supply passage 4A and a dialysis fluid discharge passage 4B in the dialysis fluid circuit.

To perform the priming procedure, in a state where a circulation path is formed by coupling tip ends of the artery passage 3B and the vein passage 3A with each other, a dialysis fluid is supplied to the blood circuit via the fluid supply passage 11, and the supply/discharge passage 12 is connected to the dialysis fluid discharge passage 4B. At the time of the blood returning procedure, the dialysis fluid is discharged through the dialysis fluid supply passage 4A into the dialysis fluid discharge passage 4B via the supply/discharge passage 12 that supplies the dialysis fluid to the blood circuit.

## Description

### BACKGROUND OF THE INVENTION

### Field of the Invention

The present invention relates to a blood purification apparatus, and more specifically, to a blood purification apparatus that makes it possible to efficiently perform a priming procedure before treatment and a blood returning procedure after the treatment.

### Description of the Related Art

There has been known a blood purification apparatus, including: a blood circuit including an artery passage and a vein passage connected to a blood purifier; a dialysis fluid circuit including a dialysis fluid supply passage and a dialysis fluid discharge passage connected to the blood purifier; a blood pump provided for the artery passage; and dialysis fluid supply/retrieval means that is provided for the dialysis fluid circuit and is for supplying and retrieving a dialysis fluid to and from the blood purifier (Japanese Patent No. 4091873, Japanese Patent No. 6685374, and Japanese Laid-Open Patent Application No. 2024-35694).

The blood purification apparatus as described above requires a priming procedure for causing a priming fluid to flow through the blood purifier and the blood circuit before blood purification treatment is performed, and a blood returning procedure for returning blood remaining in the blood purifier and the blood circuit to the patient.

The blood purification apparatus in Japanese Patent No. 4091873 described above includes a fluid supply passage provided between the dialysis fluid supply passage and the artery passage. At the time of the priming procedure, the dialysis fluid in the dialysis fluid supply passage is supplied, as the priming fluid, to the artery passage via the fluid supply passage, so as to cause the dialysis fluid to flow through the blood circuit.

Further, also at the time of performing the blood returning procedure in the blood purification apparatus disclosed in Japanese Patent No. 4091873, the dialysis fluid in the dialysis fluid supply passage is supplied to the artery passage via the fluid supply passage, so that the blood in the blood circuit is pushed out by the dialysis fluid and returned to the patient.

Further, in the blood purification apparatus disclosed in Japanese Patent No. 4091873, also at the time of replenishing fluid during treatment, the dialysis fluid in the dialysis fluid supply passage is supplied to the artery passage via the fluid supply passage, so that the dialysis fluid is replenished for the patient.

In contrast, the blood purification apparatus disclosed in Japanese Patent No. 6685374 includes a fluid supply passage provided between the dialysis fluid supply passage and the artery passage and, at the time of a priming procedure, tip ends of the artery passage and the vein passage are directly connected to each other (the seventh embodiment, FIG. 23, and FIG. 24) or a connection to the dialysis fluid discharge passage is established by attaching a Y-shaped tube (the ninth embodiment, FIG. 28, and FIG. 29).

With the configurations described above, via the fluid supply passage, the dialysis fluid in the dialysis fluid supply passage is supplied to the artery passage and is caused to flow through the blood circuit, so as to be discharged into the dialysis fluid discharge passage through the tip ends of the artery passage and the vein passage.

Further, the blood purification apparatus disclosed in Japanese Laid-Open Patent Application No. 2024-35694 includes, as a fluid supply passage, a blood return passage provided between the dialysis fluid supply passage and a position on the upstream side of a blood pump on the artery passage, and includes, as a fluid supply passage, a fluid replenishment passage provided either between the dialysis fluid supply passage and a point on the upstream side of the blood purifier that is on the artery passage positioned on the downstream side of the blood pump (a beforehand dilution scheme) or between the dialysis fluid supply passage and the vein passage positioned on the downstream side of the blood purifier (an afterward dilution scheme).

In the configuration described above, at the time of the priming procedure, the dialysis fluid is supplied through the fluid replenishment passage and is caused to flow through the blood circuit, so as to be discharged into the dialysis fluid discharge passage through the tip ends of the artery passage and the vein passage.

However, in the blood purification apparatus disclosed in Japanese Patent No. 4091873, at the time of the priming procedure, the dialysis fluid supplied to the blood circuit is caused to flow out through the tip ends of the artery passage and the vein passage. Thus, a problem is that work of medical workers may become cumbersome because, for example, a container for retrieval of the outflowing dialysis fluid is needed.

Further, as for the blood purification apparatus disclosed in Japanese Patent No. 6685374, if it is selected not to directly connect the tip ends of the artery passage and the vein passage to the dialysis fluid discharge passage at the time of the priming procedure, the blood circuit needs to be accompanied by the Y-shaped tube connectable to the passages. Thus, a problem is that costs of component materials increase.

In addition, in the blood purification apparatus disclosed in Japanese Laid-Open Patent Application No. 2024-35694, the dialysis fluid is supplied through the fluid supply passage that is used in common to the priming procedure, the blood returning procedure, and the fluid replenishment procedure; however, in practice, the dialysis fluid is supplied from different positions in the blood circuit between the blood returning procedure and the fluid replenishment procedure.

In other words, in the blood purification apparatus disclosed in Japanese Laid-Open Patent Application No. 2024-35694, in addition to the blood return passage used for supplying the dialysis fluid to the upstream side of the blood pump, the fluid replenishment passage is provided for supplying the dialysis fluid to either the artery passage or the vein passage positioned on the downstream side of the blood pump for the purpose of replenishing fluid.

In this connection, because the blood circuit is replaced for each patient, the blood circuit is removably attached to the blood purification apparatus. Thus, the passage connections between the dialysis fluid circuit and the blood circuit are established via connection ports.

The blood purification apparatus disclosed in Japanese Laid-Open Patent Application No. 2024-35694 includes connection ports provided in two locations on the dialysis fluid supply passage for the purpose of connecting the blood return passage and the fluid replenishment passage. In addition, another connection port is provided in a location on the dialysis fluid discharge passage for the purpose of discharging the fluid at the time of the priming procedure. Thus, there are three connection ports in total.

Similarly, for the blood purification apparatus disclosed in Japanese Patent No. 6685374, to discharge the fluid at the time of the priming procedure, if the tip ends of the vein passage and the artery passage are to be directly connected to the dialysis fluid discharge passage without using the Y-shaped tube, two connection ports are needed.

As described herein, when the quantity of the connection ports is increased to two or more, it is necessary to add not only the ports but also passages, open/close valves, filters, and the like. Thus, the increase in the number of component parts leads to an increase in the cost of the blood purification apparatus.

Furthermore, in the blood purification apparatuses disclosed in Japanese Patent No. 6685374 and Japanese Laid-Open Patent Application No. 2024-35694, the tip ends of the artery passage and the vein passage are connected to the connection ports for the purpose of discharging the fluid at the time of the priming procedure. In this situation, although the connection ports are sufficiently cleaned after the treatment, because the connection ports are parts open to the outside, there is a risk of being contaminated after being cleaned.

Consequently, before the connection to the patient is established to start the treatment, it is desirable to avoid at all costs connecting, to such connection ports, the tip ends of the vein passage and the artery passage that are in the already-sterilized blood circuit and connected to the patient.

In view of the problems described above, the present invention provides a blood purification apparatus that makes it possible to efficiently perform a priming procedure and a blood returning procedure and to keep the costs of the component materials and the apparatus down, while preventing the blood circuit from being contaminated before the treatment.

### SUMMARY OF THE INVENTION

More specifically, the blood purification apparatus set forth in claim 1 is a blood purification apparatus that includes a blood circuit having an artery passage and a vein passage connected to a blood purifier, a dialysis fluid circuit having a dialysis fluid supply passage and a dialysis fluid discharge passage connected to the blood purifier, a blood pump provided for the artery passage, and dialysis fluid supply/discharge means that is provided for the dialysis fluid circuit and that is for supplying and discharging a dialysis fluid for the blood purifier, the blood purification apparatus being configured to perform blood purification treatment by circulating blood from the artery passage to the vein passage via the blood purifier, characterized in that the blood purification apparatus includes:
a fluid supply passage which is connected at one end to the dialysis fluid supply passage and which is connected at another end to one of the artery passage and the vein passage; a supply/discharge passage which is connected at one end to the artery passage and which is connected at another end to the dialysis fluid supply passage and to the dialysis fluid discharge passage; and switching means for switching the supply/discharge passage between supplying and discharging the dialysis fluid to and from the blood circuit.

To perform a priming procedure before treatment, in a state where a circulation path is formed by coupling tip ends of the artery passage and the vein passage with each other, the dialysis fluid is supplied through the dialysis fluid supply passage to the blood circuit via the fluid supply passage, and the dialysis fluid is discharged from the blood circuit into the dialysis fluid discharge passage via the supply/discharge passage.

To perform a blood returning procedure after the treatment, the dialysis fluid is supplied through the dialysis fluid supply passage to the blood circuit via the supply/discharge passage so that blood is returned through the tip ends of the artery passage and the vein passage.

### Advantageous Effects of Invention

According to the invention set forth in claim 1, the fluid discharge at the time of the priming procedure is performed via the supply/discharge passage that is connected in advance to the dialysis fluid circuit and keeps being connected during the treatment. Thus, there is no need to attach and detach the tip ends of the artery passage and the vein passage of the blood circuit to and from the dialysis fluid discharge passage. Furthermore, the blood circuit does not need to be accompanied by a tube for the connection to the dialysis fluid discharge passage, either.

In addition, because the supply/discharge passage serves for both supplying the fluid and the discharging the fluid, it is possible to inhibit an increase in the quantity of the connection ports, while there is no need to connect the tip ends of the artery passage and the vein passage to connection ports.

Consequently, it is possible to efficiently perform the priming procedure and the blood returning procedure and to keep the costs of the component materials and the apparatus down, while preventing the blood circuit from being contaminated before the treatment.

### BRIEF DESCRIPTION OF THE DRAWINGS

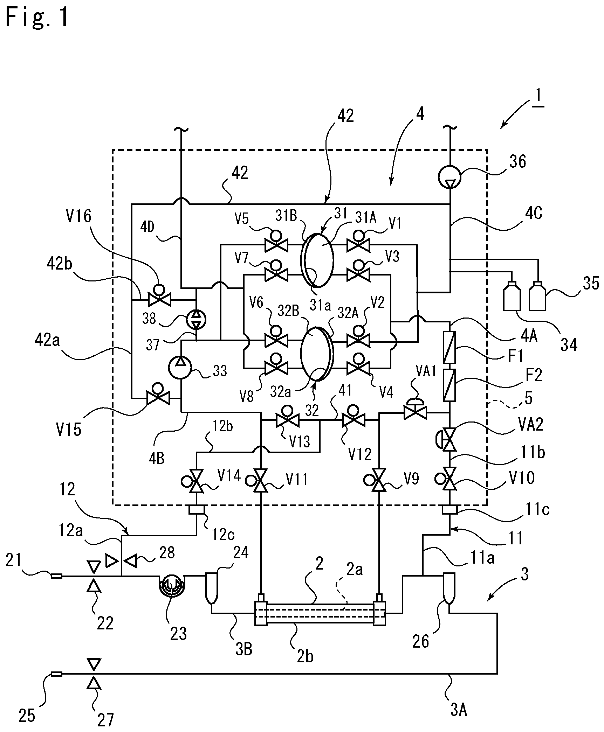

FIG. 1 is a circuit diagram of a dialysis fluid circuit and a blood circuit of a dialysis apparatus according to an embodiment of the present disclosure;

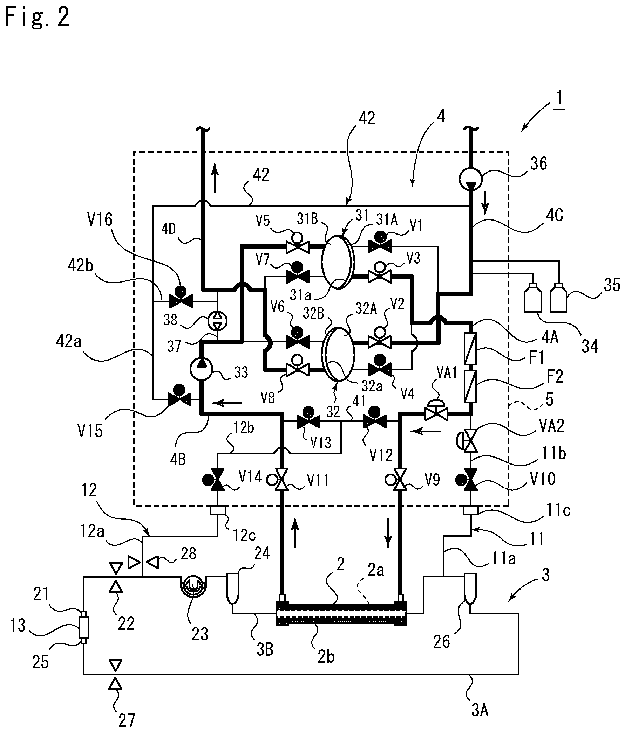

FIG. 2 is a diagram for explaining a flow of a dialysis fluid and steps in a priming procedure at the time of performing hemodialysis treatment;

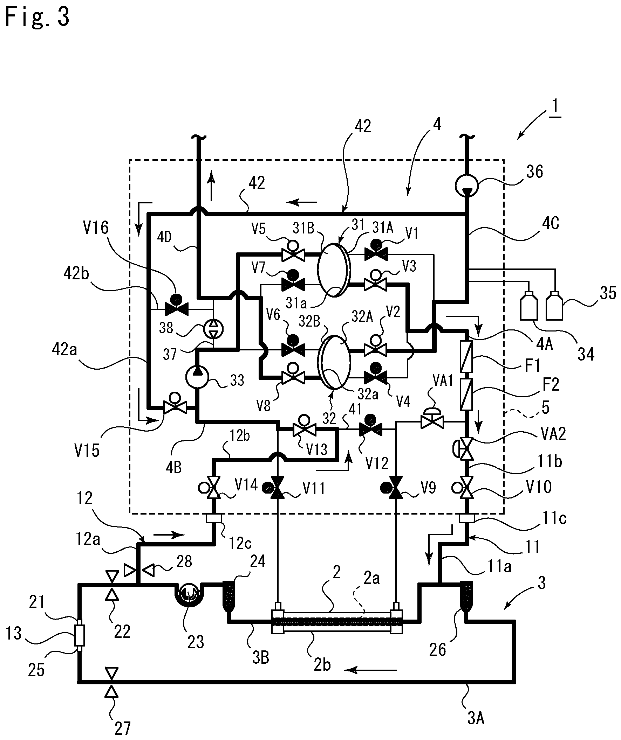

FIG. 3 is another diagram for explaining the steps in the priming procedure;

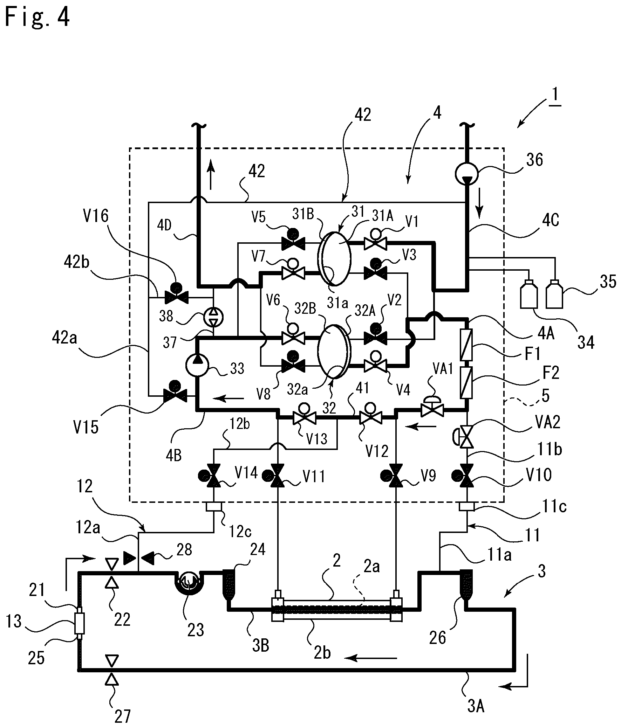

FIG. 4 is yet another diagram for explaining the steps in the priming procedure;

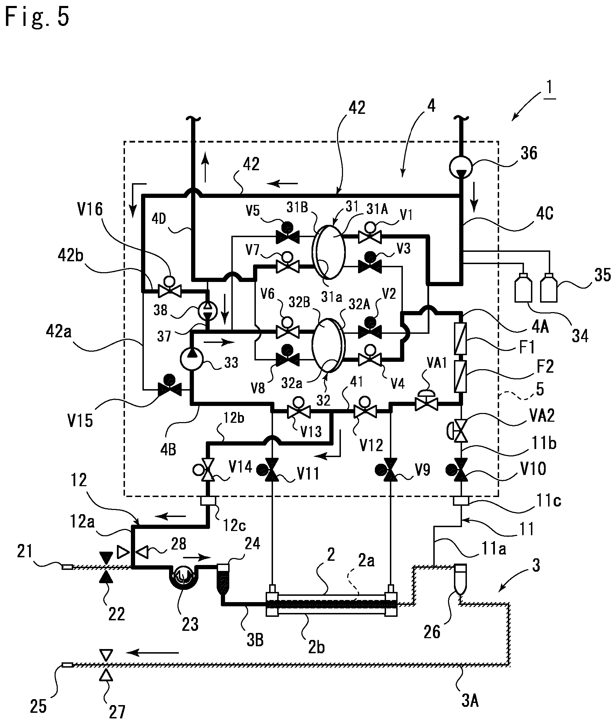

FIG. 5 is a diagram for explaining steps in a blood returning procedure;

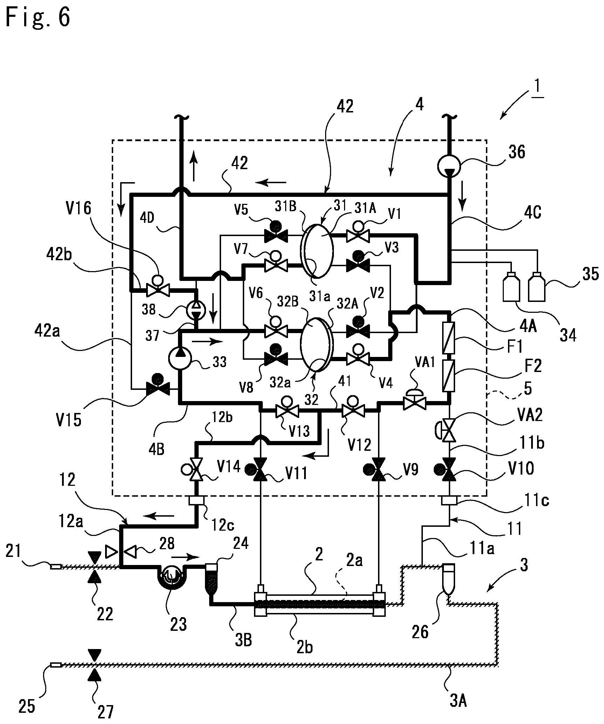

FIG. 6 is another diagram for explaining the steps in the blood returning procedure;

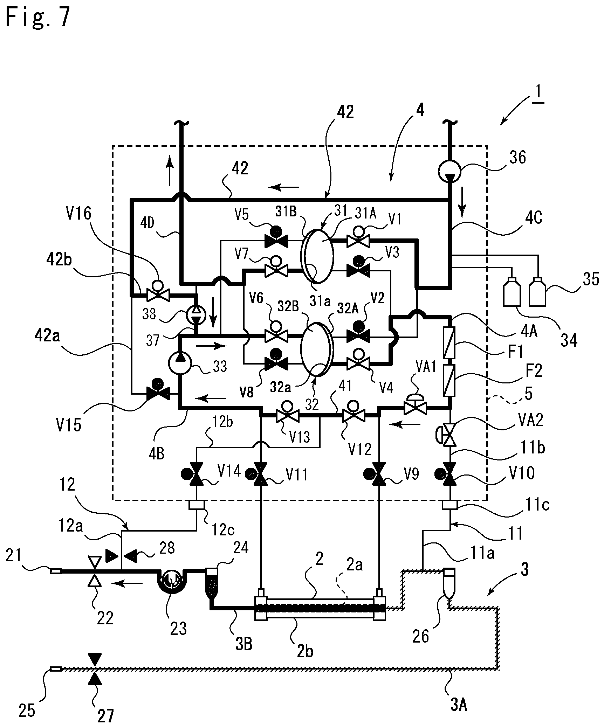

FIG. 7 is yet another diagram for explaining the steps in the blood returning procedure; and

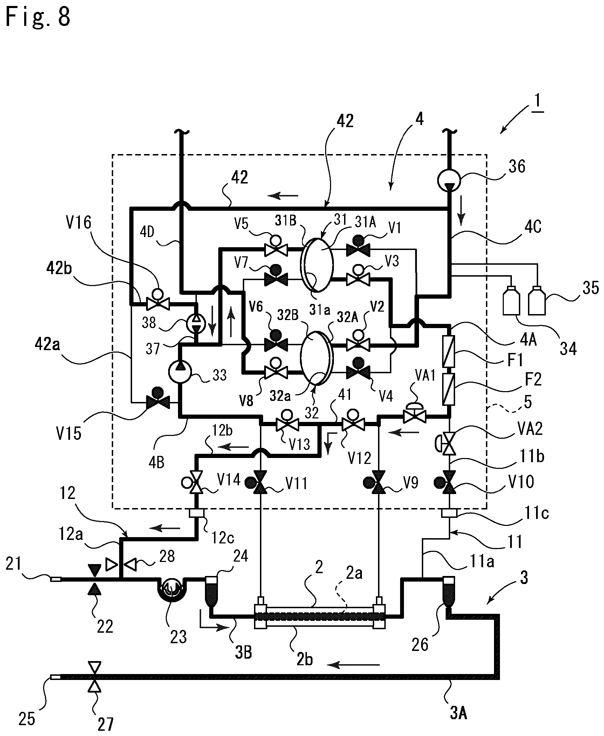

FIG. 8 is yet another diagram for explaining the steps in the blood returning procedure.

### DETAILED DESCRIPTION OF THE INVENTION

The following will describe the present invention with regard to the embodiment shown in the drawings. FIG. 1 shows a dialysis apparatus 1 that serves as a blood purification apparatus configured to perform hemodialysis being blood purification treatment and that includes a dialyzer 2 serving as a blood purifier; a blood circuit 3 connected to the dialyzer 2 and configured to cause blood to flow; and a dialysis fluid circuit 4 connected to the dialyzer 2 and configured to cause a dialysis fluid to flow. Further, the dialysis apparatus 1 is configured to be controlled by controlling means (not shown).

The dialysis apparatus 1 includes a casing 5 (shown with a broken line) that has the dialysis fluid circuit 4 built therein. Every time dialysis treatment is performed, a new dialyzer 2 and a new blood circuit 3 that have been sterilized are attached to the outside of the casing 5.

Further, before dialysis treatment, as shown in FIGS. 2 to 4, a priming procedure is performed to cause a priming fluid to flow through the dialyzer 2 and the blood circuit 3 that have been attached. In the present embodiment, the dialysis fluid flowing through the dialysis fluid circuit 4 is used as the priming fluid.

In addition, after dialysis treatment, as shown in FIGS. 5 to 8, a blood returning procedure is performed to return blood remaining in the dialyzer 2 and the blood circuit 3 since the dialysis treatment, to the patient.

The dialyzer 2 has a structure in which innumerable hollow fibers 2a serving as a blood purification membrane are accommodated inside a housing 2b having a tubular shape. The inside of the hollow fibers 2a is allowed to communicate with the blood circuit 3 so that blood flows therethrough, whereas the outside of the hollow fibers 2a within the housing 2b is allowed to communicate with the dialysis fluid circuit 4 so that the dialysis fluid flows in the direction opposite to the blood flowing direction. During the dialysis treatment, hemodialysis is performed between the blood flowing inside the hollow fibers 2a and the dialysis fluid flowing on the outside thereof.

The blood circuit 3 includes a vein passage 3A used for returning the blood from the dialyzer 2 to the patient and an artery passage 3B used for supplying blood drawn from the patient to the dialyzer 2. A fluid replenishment passage 11a structuring a fluid supply passage 11 is provided so as to branch off from the vein passage 3A. A blood return passage 12a structuring a supply/discharge passage 12 is provided so as to branch off from the artery passage 3B. These passages are each structured by using a tube made of elastic resin, such as silicone rubber.

One end of the artery passage 3B is connected to the dialyzer 2, while a drip chamber 24 is provided between the one end and a branching position of the blood return passage 12a. Provided at a tip end part of the other end is a connector 21 to which a puncture needle to puncture the patient with is attached.

Further, provided on the outside of the casing 5 are a first clamp 22 and a blood pump 23. The artery passage 3B is held by a plurality of holders (not shown) provided on the outside of the casing 5, in such a manner that the first clamp 22 is positioned between the tip end part and the branching position of the blood return passage 12a and that the blood pump 23 is positioned between the branching position of the blood return passage 12a and the drip chamber 24.

In this manner, the artery passage 3B is provided with the first clamp 22 and the blood pump 23. By pinching the tube structuring the artery passage 3B, the first clamp 22 is configured to block the flow of the fluid in the tube.

Further, the blood pump 23 is structured by using a roller pump that is rotatable in forward and backward directions, to deliver fluid by squeezing the tube structuring the artery passage 3B sequentially with a plurality of rollers. With the forward rotation, the fluid is delivered through the tip end part of the artery passage 3B toward the dialyzer 2. With the backward rotation, the fluid is delivered in the opposite direction. When the blood pump 23 is stopped, one of the rollers squeezes the tube so as to block the flow of the fluid in the tube.

One end of the vein passage 3A is connected to the dialyzer 2. Provided at a tip end part of the other end is a connector 25 to which a puncture needle to puncture the patient with is attached. A drip chamber 26 is provided between the connector 25 and a branching position of the fluid replenishment passage 11a, while being positioned close to the branching position.

Further, a second clamp 27 is provided on the outside of the casing 5. The vein passage 3A has the second clamp 27 provided between the tip end part and the drip chamber 26 and is close to the tip end.

Further, at the time of the priming procedure, as shown in FIG. 2, the connector 21 at the tip end part of the artery passage 3B and the connector 25 at the tip end part of the vein passage 3A are coupled together by each being inserted in coupling means 13 structured with a tubular member, so as to form a circulation path.

The coupling means 13 that is sterilized and new is prepared, together with the artery passage 3B and the vein passage 3A.

The fluid replenishment passage 11a structuring the fluid supply passage 11 is provided so as to branch off from a position between the connection end of the vein passage 3A to the dialyzer 2 and the drip chamber 26 and is configured to be connected to the dialysis fluid circuit 4, as a result of connecting the fluid replenishment passage 11a to a fluid supplying-purpose connection port (a fluid supply port 11c) provided on the outside of the casing 5.

The fluid replenishment passage 11a of the blood circuit 3 branches off from the vein passage 3A according to an afterward dilution scheme. However, when fluid is replenished according to a beforehand dilution scheme, the fluid replenishment passage 11a may branch off from the artery passage 3B.

The blood return passage 12a structuring the supply/discharge passage 12 is provided so as to branch off from a location between the position where the first clamp 22 is provided on the artery passage 3B and the position where the blood pump 23 is provided.

As a result of being connected to a supplying/discharging-purpose connection port (a supply/discharge port 12c) provided on the outside of the casing 5, the blood return passage 12a is connected to the dialysis fluid circuit 4.

Further, while being connected to the supply/discharge port 12c, the blood return passage 12a can be attached to a third clamp 28 provided on the outside of the casing 5. The flow of the fluid in the tube is blocked, as a result of the third clamp 28 pinching the tube structuring the blood return passage 12a.

The dialysis fluid circuit 4 includes a dialysis fluid supply passage 4A for supplying a fresh dialysis fluid to the dialyzer 2 and a dialysis fluid discharge passage 4B for retrieving used dialysis fluid that has passed through the dialyzer 2. Also, as dialysis fluid supply/retrieval means, the dialysis fluid circuit 4 includes a first dialysis fluid chamber 31 and a second dialysis fluid chamber 32 that are identical and store the dialysis fluid therein, as well as a fluid delivery pump 33 provided for the dialysis fluid discharge passage 4B. Similarly to the passages structuring the blood circuit 3, the passages structuring the dialysis fluid circuit 4 are also configured by using a tube that is made of resin and elastic.

The interior of the first and the second dialysis fluid chambers 31 and 32 are divided into two compartments by elastic membranes 31a and 32a, so as to form supply compartments 31A and 32A storing fresh dialysis fluid therein and retrieval compartments 31B and 32B storing used dialysis fluid therein.

To each of the supply compartments 31A and 32A, the dialysis fluid supply passage 4A and a water supply passage 4C connected to purified water supply means (not shown) are each branched and connected. The branched passages of the water supply passage 4C are provided with fluid supply valves V1 and V2. The branched passages of the dialysis fluid supply passage 4A are provided with supply valves V3 and V4.

Meanwhile, to each of the retrieval compartments 31B and 32B, the dialysis fluid discharge passage 4B and a fluid discharge passage 4D connected to a fluid disposal pipe (not shown) are each branched and connected. The branched passages of the dialysis fluid discharge passage 4B are provided with retrieval valves V5 and V6. The branched passages of the fluid discharge passage 4D are provided with fluid discharge valves V7 and V8.

Along the water supply passage 4C, a fluid A supply source 34 and a fluid B supply source 35 are connected for supplying undiluted fluid A and undiluted fluid B, which are undiluted fluids of the dialysis fluid, while a water supply pump 36 is provided on the upstream side thereof.

The dialysis fluid supply passage 4A is provided with a first dialysis fluid filter F1 and a second dialysis fluid filter F2 each realized with an endotoxin removal filter that purifies the dialysis fluid, as well as a first flow volume adjustment valve VA1 and a ninth open/close valve V9 controlled by the controlling means.

A fluid supply branch passage 11b connected to the fluid replenishment passage 11a and structuring the fluid supply passage 11 branches off from a location between the second dialysis fluid filter F2 and the first flow volume adjustment valve VA1. The fluid supply branch passage 11b is provided with a second flow volume adjustment valve VA2 controlled by the controlling means and a tenth open/close valve V10 serving as a fluid supply open/close valve. A tip end of the fluid supply branch passage 11b is connected to the fluid supply port 11c.

With this arrangement, at the time of replenishing fluid during the treatment, the flow volume of the dialysis fluid supplied through the dialysis fluid supply passage 4A to the dialyzer 2 and the flow volume of the dialysis fluid supplied through the fluid supply branch passage 11b to the blood circuit 3 are adjusted, by controlling opening degrees of the first flow volume adjustment valve VA1 on the dialysis fluid supply passage 4A and the second flow volume adjustment valve VA2 on the fluid supply branch passage 11b.

In this situation, the fluid supply branch passage 11b branches off from the dialysis fluid supply passage 4A on the downstream side of the first dialysis fluid filter F1 and the second dialysis fluid filter F2 and is configured to supply the dialysis fluid that has been purified to the blood circuit 3.

The dialysis fluid discharge passage 4B is provided with an eleventh open/close valve V11 controlled by the controlling means and the fluid delivery pump 33. Provided in a position adjacent to the fluid delivery pump 33 on the downstream side thereof is a water removal passage 37 positioned between the fluid delivery pump 33 and the fluid discharge passage 4D. The water removal passage 37 is provided with a water removal pump 38.

The water removal pump 38 is structured by using a so-called piston pump capable of accurately delivering fluid by a prescribed amount at a time, while the fluid delivery direction thereof can be switched between a forward direction and a backward direction. The forward direction denotes the direction in which the used dialysis fluid is delivered through the dialysis fluid discharge passage 4B to the fluid discharge passage 4D via the water removal passage 37, for the purpose of removing water at the time of the dialysis treatment. Further, by switching between the forward and the backward directions, it is possible to deliver the fluid in the opposite direction, i.e., through the fluid discharge passage 4D toward the dialysis fluid discharge passage 4B.

Also, the dialysis fluid circuit 4 is further provided with a first bypass passage 41 positioned between the dialysis fluid supply passage 4A and the dialysis fluid discharge passage 4B. The first bypass passage 41 is provided with a twelfth open/valve V12 serving as a supply open/close valve and controlled by the controlling means and a thirteenth open/close valve V13 serving as a discharge open/close valve.

One end of the first bypass passage 41 is connected to a certain point on the dialysis fluid supply passage 4A that is positioned between the first flow volume adjustment valve VA1 and the ninth open/close valve V9. The other end is connected to a certain point on the dialysis fluid discharge passage 4B that is positioned between the eleventh open/close valve V11 and the fluid delivery pump 33.

The supply/discharge branch passage 12b connected to the blood return passage 12a and structuring the supply/discharge passage 12 branches off from a certain location on the first bypass passage 41 that is positioned between the twelfth open/close valve V12 and the thirteenth open/close valve V13. The supply/discharge branch passage 12b is provided with a fourteenth open/close valve V14 controlled by the controlling means. A tip end of the supply/discharge branch passage 12b is connected to the supply/discharge port 12c.

With this arrangement, when the twelfth open/close valve V12 on the first bypass passage 41 is opened, while the thirteenth open/close valve V13 is closed, and the fourteenth open/close valve V14 on the supply/discharge branch passage 12b is opened, the blood circuit 3 communicates with the dialysis fluid supply passage 4A via the supply/discharge passage 12, and it is therefore possible to supply the dialysis fluid that has been purified to the blood circuit 3 and to perform the blood returning procedure.

On the contrary, when the twelfth open/close valve V12 is closed, while the thirteenth open/close valve V13 and the fourteenth open/close valve V14 are opened, the blood circuit 3 communicates with the dialysis fluid discharge passage 4B via the supply/discharge passage 12, so that it is possible to discharge the fluid from the blood circuit 3.

In other words, these elements structure switching means according to the present invention for switching the supply/discharge passage 12 between supplying and discharging the dialysis fluid to and from the blood circuit 3.

Further, a second bypass passage 42 is provided so as to branch off from the downstream side of the water supply pump 36 on the water supply passage 4C. The second bypass passage 42 is further branched into a first branch passage 42a and a second branch passage 42b.

The first branch passage 42a is connected to a certain location on the dialysis fluid discharge passage 4B that is between the connection position of the first bypass passage 41 and the fluid delivery pump 33. The second branch passage 42b is connected to a certain location on the water removal passage 37 that is between the water removal pump 38 and the fluid discharge passage 4D.

Further, the first branch passage 42a is provided with a fifteenth open/close valve V15 controlled by the controlling means. The second branch passage 42b is provided with a sixteenth open/close valve V16 controlled by the controlling means.

With this configuration, by opening the fifteenth open/close valve V15, it is possible to cause purified water to flow, in a large flow volume, through the water supply passage 4C into the retrieval compartments 31B and 32B while employing the fluid delivery pump 33.

Further, by opening the sixteenth open/close valve V16 and delivering the fluid after changing the fluid delivery direction of the water removal pump 38 into the opposite direction, it is possible to additionally supply, via the water removal passage 37, purified water in a small flow volume and with excellent precision to the dialysis fluid caused to flow into the retrieval compartments 31B and 32B by the fluid delivery pump 33.

A flow of the dialysis fluid during hemodialysis treatment performed by the dialysis apparatus 1 configured as described above will be explained, with reference to FIG. 2. In FIG. 2, the parts where the dialysis fluid is flowing are shown with bold lines. The open/close valves and clamps that are open are shown in white, whereas the open/close valves and clamps that are closed are shown in black.

For the first dialysis fluid chamber 31, the third open/close valve V3 and the fifth open/close valve V5 are in the open state, while the ninth open/close valve V9 and the eleventh open/close valve V11 are opened so that the dialysis fluid circuit 4 communicates with the dialyzer 2. The tenth open/close valve V10, the twelfth open/close valve V12, the thirteenth open/close valve V13, the fourteenth open/close valve V14, the fifteenth open/close valve V15, and the sixteenth open/close valve V16 are closed.

As a result, a sealed circuit is formed by the supply compartment 31A of the first dialysis fluid chamber 31, the dialysis fluid supply passage 4A, the outside of the hollow fibers 2a within the housing 2b of the dialyzer 2, the dialysis fluid discharge passage 4B, and the retrieval compartment 32B of the second dialysis fluid chamber 32.

In this state, to the supply compartment 32A of the second dialysis fluid chamber 32, the purified water, the undiluted fluid A, and the undiluted fluid B are supplied in a prescribed ratio from the purified water supply means, the fluid A supply source 34, and the fluid B supply source 35 via the water supply passage 4C after flowing through the second open/close valve V2 that is open, so as to be mixed together inside the supply compartment 32A so that fresh dialysis fluid is prepared.

In this situation, when the purified water and the undiluted fluids have flowed into the supply compartment 32A, the capacity of the supply compartment 32A increases while the elastic membrane 32a is changing the shape thereof. In conjunction therewith, the capacity of the retrieval compartment 32B decreases, so that the used dialysis fluid flows from the retrieval compartment 32B through the eighth open/close valve V8 that is open, so as to be discharged via the fluid discharge passage 4D.

In contrast, for the first dialysis fluid chamber 31, as a result of the fluid delivery pump 33 operating while the prepared fresh dialysis fluid is stored in the supply compartment 31A, the fresh dialysis fluid is delivered to the dialyzer 2 via the dialysis fluid supply passage 4A, whereas the used dialysis fluid that has passed through the dialyzer 2 is stored into the retrieval compartment 31B via the dialysis fluid discharge passage 4B. Consequently, the dialysis fluid moves from the supply compartment 31A to the retrieval compartment 31B, so that the capacity of the retrieval compartment 32B increases.

After that, when the dialysis fluid is delivered in a prescribed amount from the supply compartment 31A to the retrieval compartment 31B in the first dialysis fluid chamber 31, the open/closed states are switched between the fluid supply valves V1 and V2 and the fluid discharge valves V7 and V8, as well as the supply valves V3 and V4 and the retrieval valves V5 and V6 provided for the first and the second dialysis fluid chambers 31 and 32.

As a result, with respect to the second dialysis fluid chamber 32, the fresh dialysis fluid is supplied from the supply compartment 32A to the dialyzer 2, whereas the used dialysis fluid is retrieved into the retrieval compartment 32B. In contrast, with respect to the first dialysis fluid chamber 31, the fresh dialysis fluid is stored in the supply compartment 31A, whereas the used dialysis fluid is discharged from the retrieval compartment 31B into the fluid discharge passage 4D.

After that, by repeatedly performing the above operations alternately, it is possible to continuously supply fresh dialysis fluid to the dialyzer 2 and to retrieve used dialysis fluid, by employing the dialysis fluid circuit 4. Thus, hemodialysis is carried out by the dialyzer 2 with the blood flowing through the blood circuit 3.

Further, to perform a water removal operation so as to remove water from blood of the patient during dialysis treatment, the controlling means causes the water removal pump 38 provided for the water removal passage 37 to deliver fluid in the forward direction, so that the used dialysis fluid flowing through the dialysis fluid discharge passage 4B is discharged into the fluid discharge passage 4D via the water removal passage 37 by a water removal amount set in advance.

As a result, a sealed circuit is formed by the supply compartments 31A, 32A, the dialysis fluid supply passage 4A, the outside of the hollow fibers 2a within the housing 2b of the dialyzer 2, the dialysis fluid discharge passage 4B, and the retrieval compartments 31B, 32B, while the inside of the circuit is filled with the dialysis fluid. Accordingly, from the blood flowing through the inside of the hollow fibers 2a of the dialyzer 2, water is removed in an amount corresponding to the amount of the used dialysis fluid discharged by the water removal pump 38.

Furthermore, at the time of performing a fluid replenishment operation to replenish dialysis fluid for the patient during dialysis treatment, the controlling means controls opening degrees of the first flow volume adjustment valve VA1 on the dialysis fluid supply passage 4A and the second flow volume adjustment valve VA2 on the fluid supply branch passage 11b and opens the tenth open/close valve V10 on the fluid supply branch passage 11b, so that while fresh dialysis fluid is supplied to the dialyzer 2, fresh dialysis fluid in a prescribed amount is supplied through the dialysis fluid supply passage 4A to the blood circuit 3 via the fluid supply passage 11, so as to replenish the fluid for the patient.

Next, a priming procedure using the dialysis apparatus 1 according to the present embodiment will be explained, with reference to FIGS. 2 to 4. In the following description also, the parts where the dialysis fluid is flowing are shown with bold lines. The open/close valves and clamps that are open are shown in white, whereas the open/close valves and clamps that are closed are shown in black.

FIG. 2 shows an operation to cause the dialysis fluid to flow through an outside part of the hollow fibers 2a within the housing 2b of the dialyzer 2 that are connected to the dialysis fluid circuit 4.

To begin with, the casing 5 of the dialysis apparatus 1 is caused to support the dialyzer 2 that is new and has been sterilized. Also, one ends of the artery passage 3B and the vein passage 3A of the blood circuit 3 that is new and has been sterilized are connected to the dialyzer 2, so that the artery passage 3B and the vein passage 3A are held by and attached to a plurality of holders (not shown) on the outside of the casing 5.

Further, the tip end part of the artery passage 3B and the tip end part of the vein passage 3A of the blood circuit 3 are coupled together by the coupling means 13 that is provided for the blood circuit, is new, and has been sterilized, so as to make the blood circuit 3 a circulation path.

Furthermore, the artery passage 3B is attached to the blood pump 23 and set with the first clamp 22. A tip end of the blood return passage 12a is connected to the supply/discharge port 12c, so as to be connected to the supply/discharge branch passage 12b of the dialysis fluid circuit 4. In addition, the blood return passage 12a is set with the third clamp 28.

The vein passage 3A is set with the second clamp 27, and a tip end of the fluid replenishment passage 11a is connected to the fluid supply port 11c, so as to be connected to the fluid supply branch passage 11b of the dialysis fluid circuit 4.

Furthermore, as for the dialysis fluid circuit 4, the ninth open/close valve V9 and the eleventh open/close valve V11 are opened so as to allow communication with the dialyzer 2. The tenth open/close valve V10, the twelfth open/close valve V12, the thirteenth open/close valve V13, the fourteenth open/close valve V14, the fifteenth open/close valve V15, and the sixteenth open/close valve V16 are closed.

In that state, when an operation to start the priming procedure is performed on the controlling means, the controlling means brings the fluid delivery pump 33 into operation, so as to cause the fresh dialysis fluid stored in the supply compartment 31A of the first dialysis fluid chamber 31 in the state shown in FIG. 2 to flow into the dialysis fluid supply passage 4A, similarly to when dialysis treatment is performed.

As a result, the fresh dialysis fluid flows into the dialyzer 2 through the dialysis fluid supply passage 4A, and also, the dialysis fluid that has passed through the dialyzer 2 is discharged into the dialysis fluid discharge passage 4B. Consequently, the dialysis fluid is caused flow on the outside of the hollow fibers 2a within the housing 2b of the dialyzer 2.

Next, FIG. 3 shows an operation to cause the dialysis fluid to flow through the blood circuit 3 and an inside part of the hollow fibers 2a in the dialyzer 2 that are connected to the blood circuit 3.

Starting with the state shown in FIG. 2, the controlling means closes the ninth open/close valve V9 on the dialysis fluid supply passage 4A and the eleventh open/close valve V11 on the dialysis fluid discharge passage 4B, opens the tenth open/close valve V10 on the fluid supply branch passage 11b serving as a fluid supply open/close valve, the fourteenth open/close valve V14 on the supply/discharge branch passage 12b, the thirteenth open/close valve V13 on the first bypass passage 41, and the fifteenth open/close valve V15 on the first branch passage 42a of the second bypass passage 42, and keeps closed the twelfth open/close valve V12 serving as a supply open/close valve.

As a result, the blood circuit 3 is brought into communication with the dialysis fluid discharge passage 4B via the supply/discharge passage 12.

Further, in the blood circuit 3, the first to the third clamps 22, 27 and 28 are all opened.

In addition, while having the fluid delivery pump 33 on the dialysis fluid discharge passage 4B in operation, the controlling means adjusts the first and the second flow volume adjustment valves VA1 and VA2 so that a full volume flows into the fluid supply branch passage 11b, so as to cause all of the dialysis fluid in the dialysis fluid supply passage 4A to flow into the fluid replenishment passage 11a.

As a result, in the state shown in FIG. 3, due to the action of the fluid delivery pump 33, the purified water flows through the water supply passage 4C into the dialysis fluid discharge passage 4B via the first branch passage 42a of the second bypass passage 42, so that the purified water flows into the retrieval compartment 31B of the first dialysis fluid chamber 31.

Consequently, the fresh dialysis fluid that was stored in the supply compartment 31A of the first dialysis fluid chamber 31 is discharged, so that the dialysis fluid flows through the dialysis fluid supply passage 4A and the fluid supply passage 11 and subsequently flows into the vein passage 3A in the blood circuit 3.

Meanwhile, in the blood circuit 3, the controlling means has caused the blood pump 23 to rotate in the backward direction so that the fluid is delivered toward the tip end part of the artery passage 3B, which means that the fluid is delivered in the opposite direction as compared to during the dialysis treatment. In addition, the flow volume is set to be smaller than the flow volume into the vein passage 3A through the fluid replenishment passage 11a.

For example, when the flow volume of the dialysis fluid flowing through the fluid supply passage 11 into the fluid replenishment passage 11a is set to 600 ml/min, while the flow volume caused by the blood pump 23 is set to 300 ml/min, although the blood pump 23 causes the dialysis fluid in an amount of 300 ml/min to flow through the vein passage 3A past the dialyzer 2 to be delivered toward the tip end part of the artery passage 3B, the rest of the dialysis fluid in an amount of 300 ml/min flows toward the tip end part of the vein passage 3A.

Consequently, the dialysis fluid flowing in the opposite direction flows beyond the blood pump 23 and is discharged through the blood return passage 12a. In addition, the dialysis fluid that has flowed toward the tip end part of the vein passage 3A flows beyond the coupling means 13 coupling the tip ends of the vein passage 3A and the artery passage 3B with each other so as to be discharged through the blood return passage 12a. In this manner, the dialysis fluid is caused to flow through the entire region of the blood circuit 3 including the fluid replenishment passage 11a and the blood return passage 12a.

The dialysis fluid discharged through the blood return passage 12a in the manner described above flows through the supply/discharge branch passage 12b and, via the first bypass passage 41, flows through the dialysis fluid discharge passage 4B so as to be retrieved into the retrieval compartment 31B of the first dialysis fluid chamber 31.

As a result of the dialysis fluid being discharged from the blood circuit 3 to the dialysis fluid discharge passage 4B through the supply/discharge passage 12 in the manner described above, there is no need to perform the fluid discharging process, and also, component materials such as the Y-shaped tube become unnecessary, as compared to the example in which the dialysis fluid is discharged through the tip ends of the vein passage 3A and the artery passage 3B.

Furthermore, to discharge fluid from the blood circuit 3, there is no need to directly connect the connectors 21 and 25 at the tip end parts of the vein passage 3A and the artery passage 3B to a fluid discharging-purpose connection port or to allow the communication to the dialysis fluid discharge passage 4B via a Y-shaped tube or the like. Instead, the tip end parts of the vein passage 3A and the artery passage 3B are coupled together by the coupling means 13 that has been sterilized. Consequently, there is no risk of compromising purity.

FIG. 4 shows an operation to cause the dialysis fluid to circulate in the blood circuit 3 and to remove air bubbles from the inside of the blood circuit 3 by using the drip chambers 24 and 26.

Starting with the state shown in FIG. 3, the controlling means closes the tenth open/close valve V10 on the fluid supply branch passage 11b and the fourteenth open/close valve V14 on the supply/discharge branch passage 12b and opens the twelfth open/close valve V12 on the first bypass passage 41. Further, the controlling means closes the third clamp 28 on the blood return passage 12a.

In the dialysis fluid circuit 4, although the dialysis fluid is supplied and discharged by the first and the second dialysis fluid chambers 31 and 32 similarly to during the dialysis treatment, the dialysis fluid in the dialysis fluid supply passage 4A does not pass through the dialyzer 2, but flows into the dialysis fluid discharge passage 4B via the first bypass passage 41.

In contrast, in the blood circuit 3, the controlling means causes the blood pump 23 to rotate either in the forward direction or the backward direction, so as to cause the dialysis fluid to circulate in the blood circuit 3 forming a circulation path. As a result, the air bubbles contained in the dialysis fluid are removed by the drip chambers 24 and 26, and the priming procedure is thus completed.

When the priming procedure is completed in this manner, a medical worker separates the vein passage 3A and the artery passage 3B from the coupling means 13, attaches the puncture needles to the tip end parts so as to puncture the patient therewith, and is thus able to start dialysis treatment.

Next, a blood returning procedure after dialysis treatment will be explained, with reference to FIGS. 5 to 8. In these drawings also, the flow of the dialysis fluid is shown with bold lines. The blood remaining in the blood circuit 3 is shown with hatching.

As shown in FIG. 5, when the dialysis treatment is finished and the controlling means is instructed to start the blood returning procedure, the controlling means closes the tenth open/close valve V10 serving as a fluid supply open/close valve, further blocks the communication with the dialyzer 2 by closing the ninth open/close valve V9 on the dialysis fluid supply passage 4A and the eleventh open/close valve V11 on the dialysis fluid discharge passage 4B, and opens the twelfth open/close valve V12 serving as a supply open/close valve on the first bypass passage 41 and the thirteenth open/close valve V13 serving as a discharge open/close valve.

As a result, in the state shown in FIG. 5, the fourth open/close valve V4 of the second dialysis fluid chamber 32 is opened so that the supply compartment 32A is allowed to communicate with the dialysis fluid supply passage 4A, while the sixth open/close valve V6 is opened so that the dialysis fluid discharge passage 4B is allowed to communicate with the retrieval compartment 32B, so that a sealed circuit is formed by the supply compartment 32A, the dialysis fluid supply passage 4A, the first bypass passage 41, the dialysis fluid discharge passage 4B, and the retrieval compartment 32B. When the fluid delivery pump 33 is operated in this state, the dialysis fluid moves from the supply compartment 32A into the retrieval compartment 32B.

In that state, while keeping the fluid delivery pump 33 in operation, the controlling means opens the sixteenth open/close valve V16 provided for the second branch passage 42b of the second bypass passage 42 and causes the water removal pump 38 on the water removal passage 37 to deliver fluid in the opposite direction, so that purified water is supplied by a prescribed amount at a time through the second bypass passage 42 via the water removal passage 37 into the dialysis fluid discharge passage 4B. At the same time, the fourteenth open/close valve V14 provided for the supply/discharge branch passage 12b and the third clamp 28 configured to open/close the blood return passage 12a are opened.

Accordingly, the purified water that has flowed in through the water removal passage 37 is delivered by the fluid delivery pump 33, so as to flow through the dialysis fluid discharge passage 4B into, in the state shown in FIG. 5, the retrieval compartment 32B of the second dialysis fluid chamber 32.

As a result, because a sealed circuit is formed by the supply compartment 32A of the second dialysis fluid chamber 32, the dialysis fluid supply passage 4A, the first bypass passage 41, the dialysis fluid discharge passage 4B, and the retrieval compartment 32B, a certain part of the dialysis fluid supplied through the dialysis fluid supply passage 4A in an amount equal to the amount of the purified water delivered by the water removal pump 38 overflows from the sealed circuit and flows through the first bypass passage 41 via the supply/discharge branch passage 12b and the blood return passage 12a into the artery passage 3B of the blood circuit 3.

Meanwhile in the blood circuit 3, as shown in FIG. 5, the controlling means is configured to cause the blood pump 23 to rotate in the forward direction while the first clamp 22 is closed and the second clamp 27 is opened, so as to deliver the dialysis fluid flowing in through the blood return passage 12a toward the dialyzer 2 by a prescribed amount.

As a result, in the example shown in FIG. 5, the dialysis fluid that has flowed into the artery passage 3B through the blood return passage 12a is delivered by the blood pump 23 and flows toward the dialyzer 2, so as to fill the inside of substantially all the hollow fibers 2a in the dialyzer 2.

In the vein passage 3A beyond the dialyzer 2, because the second clamp 27 is opened, blood is returned to the patient through the tip end part of the vein passage 3A, in an amount corresponding to the amount of the dialysis fluid that has flowed into the artery passage 3B.

In contrast, because the first clamp 22 is closed on the tip end part side (the patient side) relative to the branching position of the blood return passage 12a from the artery passage 3B, the state is maintained in which blood remains without an inflow of the dialysis fluid.

To cope with the above situation, subsequent to the state shown in FIG. 5, after the second clamp 27 is closed as shown in FIG. 6, the dialysis fluid in a small amount set in advance is supplied through the blood return passage 12a, so as to be delivered by the blood pump 23 in the forward direction. After that, the blood pump 23 is stopped.

As a result, the dialysis fluid in the small amount is pressed in from the blood pump 23 in the direction toward the dialyzer 2. The small amount of the dialysis fluid that is pressed in at that time refers to an amount that can be absorbed by an inflation of the elastic tube forming the passage without imposing unnecessary pressure on the artery passage 3B or the vein passage 3A and without causing the tube to come off or leak the fluid.

For example, while the second clamp 27 shown in FIG. 5 is opened, when the inflow volume through the blood return passage 12a is 100 ml, the volume to be pressed in after the closure may be 4 ml.

FIG. 7 shows an operation to return, to the patient, the blood present on the tip end part side relative to the branching position of the blood return passage 12a from the artery passage 3B.

Starting with the state shown in FIG. 6, i.e., the state in which the dialysis fluid in the prescribed small amount (e.g., 4 ml) has been pressed in by the blood pump 23 in the direction toward the dialyzer 2, the third clamp 28 on the blood return passage 12a is closed, and the first clamp 22 on the artery passage 3B is opened.

In that state, the controlling means causes the blood pump 23 to rotate in the backward direction by the same prescribed small amount, so as to cause the dialysis fluid that was pressed in from the blood pump 23 toward the dialyzer 2 to flow in the opposite direction, so that the blood remaining on the tip end side relative to the branching position of the blood return passage 12a from the artery passage 3B is returned to the patient by a prescribed small amount.

The amount of the blood remaining in the section from the branching position to the tip end part is larger than the amount of the dialysis fluid corresponding to the abovementioned prescribed small amount. Thus, the operation of returning blood by the small amount at a time is repeatedly performed multiple times.

In other words, as shown in FIG. 5, while the second clamp 27 is in an open state, the dialysis fluid in the prescribed small amount is again caused to flow in through the blood return passage 12a, so as to be delivered in the forward direction by the blood pump 23.

Subsequently, blood is returned through the tip end part of the vein passage 3A in an amount corresponding to the small amount of dialysis fluid that has flowed in. After that, while the second clamp 27 is closed as shown in FIG. 6, a prescribed small amount of dialysis fluid is caused to flow in through the blood return passage 12a, so as to be delivered in the forward direction by the blood pump 23, which is then stopped.

After that, while the first clamp 22 is opened as shown in FIG. 7, the blood pump 23 is caused to rotate in the backward direction, so as to cause the dialysis fluid in the same small amount to flow in the opposite direction, so that blood is returned to the patient through the tip end part of the artery passage 3B.

In this manner, as a result of repeatedly performing the operations in the states shown in FIGS. 5 to 7 multiple times, the blood which remained in the artery passage 3B in the section from the tip end to the blood pump 23 has been returned to the patient.

FIG. 8 shows an operation to completely return the blood in the vein passage 3A to the patient.

Starting with the state shown in FIG. 7, the controlling means opens, similarly to the state shown in FIG. 5, the fourteenth open/close valve V14 on the supply/discharge branch passage 12b and the third clamp 28 on the blood return passage 12a, and further closes the first clamp 22 on the artery passage 3B and opens the second clamp 27 on the vein passage 3A.

In that state, the controlling means has caused the blood pump 23 to rotate in the forward direction so that the dialysis fluid that has flowed in through the blood return passage 12a is delivered toward the dialyzer 2 and the vein passage 3A. Accordingly, the blood remaining in the vein passage 3A is delivered to the tip end part side, so as to be returned to the patient.

In this situation, the blood remaining in the blood circuit 3 is returned to the patient, while the controlling means is controlling the fluid delivery amounts of the water removal pump 38 and the blood pump 23, in accordance with the capacities of the inside of all the hollow fibers 2a in the dialyzer 2 and the insides of the passages such as the artery passage 3B and the vein passage 3A.

As described above, in the dialysis apparatus 1 according to the present embodiment, for the priming procedure, the circulation path is formed by coupling the connector 21 at the tip end part of the artery passage 3B in the blood circuit 3 with the connector 25 at the tip end part of the vein passage 3A by using the coupling means 13 so as to prime the dialyzer 2 and the blood circuit 3 by supplying the dialysis fluid through the fluid supply passage 11. To discharge the fluid at the time of the priming procedure, the discharging is carried out through the supply/discharge passage 12 into the dialysis fluid discharge passage 4B. It is therefore possible to perform the priming procedure without the need to connect the tip end parts of the artery passage 3B and the vein passage 3A in the blood circuit 3 to the dialysis fluid discharge passage 4B.

In addition, for the blood returning procedure, because the dialysis fluid is caused to flow into the artery passage 3B through the blood return passage 12a, there is no need to provide a separate fluid supply port for the blood returning purpose.

Further, the above embodiments were explained regarding a so-called personal dialysis apparatus configured to prepare the dialysis fluid in the first and the second dialysis fluid chambers 31 and 32; however, the present disclosure is also applicable to a so-called dialysis monitoring apparatus configured to supply a fresh dialysis fluid prepared in advance through the water supply passage 4C.

### Reference Signs List

1: dialysis apparatus
2: dialyzer
3: blood circuit
3A: vein passage
3B: artery passage
4: dialysis fluid circuit
4A: dialysis fluid supply passage
4B: dialysis fluid discharge passage
11: fluid supply passage
12: supply/discharge passage
13: coupling means
23: blood pump

## Claims

1. A blood purification apparatus that includes a blood circuit having an artery passage and a vein passage connected to a blood purifier, a dialysis fluid circuit having a dialysis fluid supply passage and a dialysis fluid discharge passage connected to the blood purifier, a blood pump provided for the artery passage, and dialysis fluid supply/discharge means that is provided for the dialysis fluid circuit and that is for supplying and discharging a dialysis fluid for the blood purifier, the blood purification apparatus being configured to perform blood purification treatment by circulating blood from the artery passage to the vein passage via the blood purifier, **characterized in that** the blood purification apparatus comprises:
a fluid supply passage which is connected at one end to the dialysis fluid supply passage and which is connected at another end to one of the artery passage and the vein passage;
a supply/discharge passage which is connected at one end to the artery passage and which is connected at another end to the dialysis fluid supply passage and to the dialysis fluid discharge passage; and
switching means for switching the supply/discharge passage between supplying and discharging the dialysis fluid to and from the blood circuit, wherein
to perform a priming procedure before treatment, in a state where a circulation path is formed by coupling tip ends of the artery passage and the vein passage with each other, the dialysis fluid is supplied through the dialysis fluid supply passage to the blood circuit via the fluid supply passage, and the dialysis fluid is discharged from the blood circuit into the dialysis fluid discharge passage via the supply/discharge passage, and
to perform a blood returning procedure after the treatment, the dialysis fluid is supplied through the dialysis fluid supply passage to the blood circuit via the supply/discharge passage, so that blood is returned through the tip ends of the artery passage and the vein passage.

2. The blood purification apparatus according to claim 1, **characterized by** comprising:
a bypass passage which is connected at one end to the dialysis fluid supply passage and which is connected at another end to the dialysis fluid discharge passage, wherein
the switching means includes a supply open/close valve that opens and closes the bypass passage and a fluid supply open/close valve that opens and closes the fluid supply passage, the one end of the supply/discharge passage is connected to a section from the tip end of the artery passage to the blood pump, whereas the other end of the supply/discharge passage is connected to a section from the supply open/close valve on the bypass passage to the other end connected to the dialysis fluid discharge passage,
to perform the priming procedure, the fluid supply open/close valve is opened while the supply open/close valve is closed, and
to perform the blood returning procedure, the supply open/close valve is opened, while the fluid supply open/close valve is closed.
